# EUROPEAN PATENT SPECIFICATION

(11) **EP 1 945 235 B1**
(45) Date of publication and mention of the grant of the patent: **26.09.2012**
(21) Application number: 06799717.1
(22) Date of filing: 02.10.2006
(51) Int. Cl.: A61K 35/74, A61P 3/04, C12N 1/20, C12R 1/23, C12R 1/245

(54) **PROBIOTICS TO INFLUENCE FAT METABOLISM AND OBESITY**
PROBIOTIKA ZUR BEEINFLUSSUNG DES FETTSTOFFWECHSELS UND VON FETTSUCHT
DES PROBIOTIQUES POUR INFLUENCER LE MÉTABOLISME DES GRAISSES ET L'OBÉSITÉ

(30) Priority: 07.10.2005 SE 0502214
(43) Date of publication of application: 23.07.2008
(60) Divisional application: 11191774.6
(73) Proprietor: Arla Foods Amba, 8260 Viby J (DK)
(72) Inventor: OHLSON, Kajsa, S-181 47 Lidingö (SE); MAHLAPUU, Margit, S-413 08 Göteborg (SE); SVENSSON, Ulla, S-223 61 Lund (SE)
(74) Representative: Plougmann & Vingtoft A/S
(86) International application number: PCT/SE2006/001117
(87) International publication number: WO 2007/043933

(56) References cited:
- EP-A- 1 177 794
- WO-A-2009/071086
- WO-A1-99/29833
- WO-A1-2004/014403
- US-B2- 6 808 703
- OHLSON KAJSA ET AL: "Lactobacillus F19: A probiotic strain suitable for consumer products" MICROBIAL ECOLOGY IN HEALTH AND DISEASE, vol. 14, no. Supplement 3, 2002, pages 27-32, XP009121473 ISSN: 0891-060X
- SULLIVAN ASA ET AL: "Lactobacillus acidophilus, Bifidobacterium lactis and Lactobacillus F19 prevent antibiotic-associated ecological disturbances of Bacteroides fragilis in the intestine." JOURNAL OF ANTIMICROBIAL CHEMOTHERAPY, vol. 52, no. 2, August 2003 (2003-08), pages 308-311, XP002541718 ISSN: 0305-7453
- MATTO J ET AL: "Intestinal survival and persistence of probiotic Lactobacillus and Bifidobacterium strains administered in triple-strain yoghurt" INTERNATIONAL DAIRY JOURNAL, ELSEVIER APPLIED SCIENCE, BARKING, GB, vol. 16, no. 10, 1 October 2006 (2006-10-01), pages 1174-1180, XP024963286 ISSN: 0958-6946 [retrieved on 2006-10-01]
- NERSTEDT ANNIKA ET AL: "Administration of Lactobacillus evokes coordinated changes in the intestinal expression profile of genes regulating energy homeostasis and immune phenotype in mice" BRITISH JOURNAL OF NUTRITION, vol. 97, no. 6, June 2007 (2007-06), pages 1117-1127, XP002541719 ISSN: 0007-1145
- TABUCHI M. ET AL.: 'Antidiabetic Effect of Lactobacillus GG in Streptozotocin-induced Diabetic Rats' BIOSCI. BIOTECHNOL. BIOCHEM. vol. 67, no. 6, 2003, pages 1421 - 1424, XP003009317
- KALAVATHY R. ET AL.: 'Effects of Lactobacillus cultures on growth performance, abdominal fat deposition, serum lipids and weight of organs of broiler chickens' BRITISH POULTRY SCIENCE vol. 44, 2003, pages 139 - 144, XP003009318
- MATSUZAKI T. ET AL.: 'Prevention of onset in an insulin-dependent diabetes mellitus model, NOD mice, buy oral feeding of Lactobacillus casei' APMIS vol. 105, no. 8, 1997, pages 643 - 649, XP003009319

## Description

### Field of the Invention

The invention relates to use of probiotic bacteria for the manufacture of a food product, feed product, dietary supplement, nutritional product, natural remedy, pharmaceutical active formulation and medicinal product to be used for controlling weight gain, preventing obesity, increasing satiety, prolonging satiation, reducing food intake, reducing fat deposition, improving energy metabolism, enhancing insulin sensitivity, treating obesity and treating insulin insensitivity.

### Background of the Invention

The interest in the use of food to influence health is rapidly increasing among researchers, health professionals and in the food industry. The metabolic syndrome including obesity, abdominal fat storage and type II diabetes is one of the main threats to human health and well-being. Today, more people are dying from eating too much than the number of people dying from eating too little. Thus, the possibility to lower the risk of obesity and body fat storage is of great value in a worldwide health perspective and the possibility to develop food products in this area is of great interest to the food industry.

The food industry has already responded to this demand by developing products with low energy density, e.g. low fat and low carbohydrate products. The possibilities to find bioactive components, naturally occurring in food or possible to add, that influence the risk of obesity, fat metabolism, glucose metabolism and satiety opens new possibilities for the industry to develop i.e. functional foods with documented health effects. Recently, studies showing a correlation between the consumption of high levels of calcium in milk and a low body mass index (BMI) have been published. Today, the conclusion that calcium in milk, and milk products, lowers the risk for obesity, body fat storage and insulin resistance has been drawn in a number of review articles. Calcium by itself does not seem to have those properties and it has been speculated that in addition to calcium there is a need for bioactive components present in the milk.

Probiotics are defined by ILSE (International life science institute) as added "microorganisms with a beneficial effect on health". Probiotics are commonly used in foods, feed products and dietary supplements for their influence on gut health, infection and the immunsystem. Some probiotic strains, but not all studied, have also been shown to influence blood cholesterol level and recently the possibility to lower blood pressure by the use of milk products fermented by proteolytic lactic acid bacteria has been found. Thus, the interest in probiotics is wide and the central role of the gut flora for gut health and well-being and the immune system is strongly established.

During the last years techniques, often referred to as genomics and nutrigenomics, have been developed. Those techniques allow new unique possibilities to study the influence of foods, nutritional components and probiotics on health and well-being. The techniques allow the study on gene-expression of thousands of genes simultaneously. Thus, new and unexpected areas for health effects can be found and the mechanism behind a health effect can be suggested.

The basis for this application is studies on man and mice interestingly showing that a probiotic product influences satiety, decreases fat storage and the risk of obesity. Possible mechanisms behind the observed effect were explained from gene-expression studies after consumption of the probiotic products.

### Description of background art

Metabolic functions contributing to overweight, the metabolic syndrome and risk of diabetes type II have been described and also the link between general nutrition and those diseases is well known. However, there is a need for diet based intervention studies to establish the role of individual foods and food group in diminishing the risk to develop those diseases. In studies in this area there has been a focus on energy density, fat and carbohydrates.

An important factor in the metabolic syndrome is the glucose metabolism including the insulin sensitivity. There is a link between body fat deposition, insulin insensitivity and overweight. There is also a link to satiety and satiation. The interactions and relationships between all those functions are complex and it is difficult to identify the exact sequence of events leading to health or disease.

A number of genes and their corresponding proteins that are involved in energy homeostasis have been described. Adipose tissue synthesizes a number of proteins with structural resemblance to cytokines and therefore these proteins are named adipokines. Some of those are also synthesised in the gut or the synthesis is regulated from the gut. The nuclear receptor PPAR-gamma is involved in the synthesis of e.g. adiponectine and the synthesis is associated with calorie intake and leptin signalling. Increased synthesis of adiponectine improves insulin sensitivity by increasing fatty-acid transport, oxidation and dissipation in skeletal muscle and by increasing the sensitivity of the hepatocyte to insulin. Another nutritionally regulated adipocyte-derivied factor influencing insulin sensitivity is resistin. Circulating resistin levels are increased in obesity while treatment of mice with resistin resulted in increased glucose production and impaired insulin action. Thus, resistin has a function in glucose homeostasis, and acts as an antagonist to insulin action, giving rise to insulin resistance. Resistin-like proteins are also expressed in the gastrointestinal tract (14). The protein apolipoprotein A-IV is secreted by the small intestine in humans and in rodents. The synthesis is stimulated by fat intake and the protein is probably involved in inhibiting food intake after ingestion of fat. Studies have shown that apolipoprotein A-IV probably is involved both in the short-time and long-time regulation of food intake.

Hooper et al (11) studied the gene expression in the gut of mice exposed to *Bacteroides thetraiotaomicron.* They concluded that the experiments show the essential role for the interaction between gut microflora and the host. No effects on energy metabolism or obesity were reported. The influence of probiotics on the gene expression in the small bowel mucosa has been studied for *Lactobacillus rhamnosus* GG (8). Their conclusion is that the administration of Lactobacillus GG is associated with a complex genetic response. The main responses seen are effects on the immune system, inflammation, apoptosis, cellgrowth and differentiation, cell adhesion and signal transcription and transduction. No effects on energy, fat, glucose or insulin metabolism are reported. In an oral presentation by W. de Vos, at the 4^{th} NIZO Dairy conference in 2005, the influence of lactobacilli e.g. *L. plantarum* 299v on gene expression in the gut was presented and the conclusion was that the effect on gene expression from different strains varies considerably. No effect on energy or fat metabolism was reported.

A few articles linking gut flora to weight gain and fat storage have been published. Bäckhed et al (7) studied the importance of the normal gut flora for weight gain and body fat storage. It was shown that gnotobiotic mice conventionalized with a normal microbiota increased their body fat with 60% and increased insulin resistance during a 14 day feeding period despite a reduced food intake. A suggestion for how the microbiota can influence triglyceride storage is presented. Thus, it is known that the normal gut microbiota influences fat metabolism, however, prior to this investigation it was not known whether the normal flora could be modified concerning fat metabolism.

Tabuchi et al. (17) describes that Lactobacillus GG cells improve the glucose tolerance in diabetic rats. This antidiabetic effect of the GG cells might be due to prevention of a decrease in insulin secretion.

Kalavathy et al (18) demonstrates that supplemention of a mixture of 12 Lactobacillus strains to the diet of proiler chickens improved the body weight gain and feed conversion rate and was effective in reducing abdominal fat deposition, serum total cholesterol, low density lipoprotein (LDL) cholesterol and triglycerides.

Matsuzaki et al (19) describes that oral feeding of Lactobacillus casei to an insulin-dependent diabetes mellitus model, NOD mice inhibited the occurrence of diabetes and regulated the host immune response.

Ohlson et al (20) reviews studies of the strain Lactobacillus F 19 and discusses how suitable it would be for consumer products.

Sullivan et al (21) studied the effect of clindamycin on the intestinal micro flora in subjects ingesting yogurt with added probiotic microorganisms. They found that Lactobacillus acidophilus, Bifidobacterium lactis and Lactobacillus F19 prevent antibiotic-associated ecological disturbances of Bacteroides fragilis in the intestine.

Ali et al (2, 3) has studied the influence of soybean isoflavones with or without probiotics to influence the cholesterol metabolism and fat deposition and find that the probiotics used had no influence on cholesterol or fat deposition. Other investigations ref. have shown that some probiotic strains may influence the level of blood cholesterol (1, 13). This and other studies where probiotic strains are compared shows that different probiotic strains have different characters and it is important to choose the right strains for specific health effects.

In the PCT patent application WO 04/014403 (15), the use of probiotics to reduce the amount of monosaccharides and disaccharides in food, and thereby treat or prevent the risk of obesity, are described. In the US patent no. 6 696 057, Bojrab (4) has patented a composition of yoghurt bacteria e.g. *Lactobacillus bulgaricus* and *Streptococcus thermophilus* for the treatment of gastrointestinal disorders, hyperlipidemia and autoimmune diseases, and in the US patent 6 641 808 Bojrab (5) describes the use of the same composition also for the treatment of obesity. This invention is also described in a European patent application 1 177 794 (6). These patents/applications have a long description on the influence of probiotics on gut health, infection and immunity but the only experiment presented that confirms the influence on obesity is a single feeding trial on one person. The use of green tea or green tea extracts in combination with a number of bioactive components and probiotics for weight loss has been patented by Gorsek in US patents 6 383 482 (9) and 6 565 847 (10). In both patents, the main active components are the green tea and the bioactive components, while probiotics are added as part of the basic composition. In the US patent 6 808 703 and the Japanese patent application 2001-292728 (16), a way to prepare a food for obesity treatment containing yeasts, enterobacteria, lactic acid bacteria, oligosaccharides and vegetable fibre is described. No examples or claims concerning treatment of obesity are presented.

### Description of the invention

An object of the invention is use of probiotic bacteria for the manufacture of a food product, feed product, dietary supplement, nutritional product, natural remedy, pharmaceutical active formulation and medicinal products to be used for controlling weight gain, preventing obesity, increasing satiety, prolonging satiation, reducing food intake, reducing fat deposition, improving energy metabolism, enhancing insulin sensitivity, treating obesity and treating insulin insensitivity.

This object is achieved in that the probiotic bacteria being selected from the group consistion of lactic acid bacteria specially *Lactobacillus casei, Lactobacillus acidophilus,* and *Bifidobacterium lactis,* and more preferably *Lactobacillus casei* F19 (LMG P-17806), *Lactobacillus acidophilus* NCFB 1748, and *Bifidobacterium lactis* Bb12 (DSM 15954) (the bacteria are deponated at LMG culture collection in Gent, available from the NCFB culture collection and from Chr. Hansen Company DK, respectively). The strains will be referred to as LMG P-17806, NCFB 1748 and Bb12 (DSM 15954) below.

*Lactobacillus casei* F19 (LMG P-17806), *Lactobacillus acidophilus* NCFB 1748, and *Bifidobacterium lactis* Bb12 (DSM 15954) are well characterised and the LMG P-17806 strain and its use is previously patented (WO 99/29833). The three strains survive well in food products and during the passage through the gut.

The impact of the invention is of considerable importance since the common lifestyle of today often means excess food intake leading to increased risk for the metabolic syndrome including obesity, abdominal fat deposition and type II diabetes. Type II diabetes is caused by a low sensitivity to insulin or insulin resistance. This threat to human health increases continuously in all age groups all over the world. Lactobacilli as probiotics are accepted as a food product and feed product, natural product, dietary supplements, and in natural remedies, pharmaceutical active formulations and medicinal products. They are easy to use in those delivery systems and can easily be consumed on a daily basis. The regular consumption of those bacteria will help to control weight gain, prevent obesity, increase satiety, prolong satiation, reduce food intake, reduce fat deposition, improve energy metabolism, enhance insulin sensitivity, treat obesity and treat insulin insensitivity and thereby the risk for diseases included in the metabolic syndrome.

Included in the invention are the following studies:
A study has promisingly shown that a probiotic product can lower the weight gain in humans. Further studies have shown that a man given probiotics reported a higher satiety and had a longer satiation than individuals given the same acidified milk without probiotics or placebo. Mice given probiotics had a lower total energy intake, less fat deposition in adipose tissue, and a lower total weight gain than individuals not given probiotics. Gene expression studies confirm that the probable mechanism behind the observed effects is an influence on a cluster of genes involved in energy, fat, sugar and insulin metabolism and on satiation.

### Examples

### 1) Weight regulation in humans

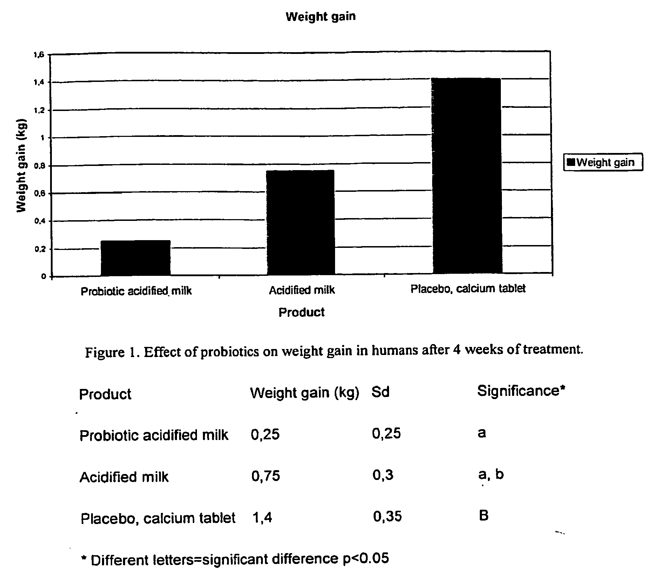

In a double blind placebo controlled study, humans were given either an acidified milk containing probiotics (LMG P-17806 and NCFB 1748) or the same acidified milk without probiotics or a calcium tablet. The groups consisted of 15, 14 and 10 persons respectively. The probiotic bacteria were added to the product in final concentration 5x10E7 CFU/ml and the number of bacteria was stable in the product during the experimental time. The participants were eating 2x2.5 dl of the product each day. The persons included were slightly overweight (BMI 25-30). The aim of the trial was to study effects of probiotics on cholesterol metabolism and weight gain was measured as a control measure. No advice concerning weight control was given but all the participants were offered a weight reduction treatment after the trial. The bacteria survived the passage in the gut and increased the number of lactobacilli found in faeces from the participants by a factor 5. After 4 weeks there was a surprising but significant difference in weight gain between the three groups. The main figure for weight gain was 0,25 kg in the group receiving the probiotic product, 0,75 kg in the group receiving the acidified milk without probiotics and 1,4 kg in the group receiving the tablet. See Figure 1.

### 2) Satiety and satiation in humans

In a double blind placebo controlled study, humans were given either an acidified milk containing probiotics in two different dosages or a placebo no 1 consisting of the same acidified milk without probiotics or a placebo no 2 consisting of acidified milk without any lactic acid bacteria at all. The probiotic microorganisms, LMG P 17806, NCFB 1748, and Bb12 (DSM15954), were added to the product in final concentration 1x10E6 CFU/ml and 5x10E7 CFU/ml of each strain. After a controlled standard breakfast, including 3 dl of any of the four products, the participants were asked to indicate satiety and hunger on a VAS-scale (Visual Analogue Scale) directly after the meal and continuously every half hour for 4 hours. A total of 10 persons were included in the study and every participant was given all four products but at different occasions and the relative differences were calculated. Directly after the meal there was a slight difference in the satiety scores between the products. The acidified milk with probiotics in concentration 5x 10E7 CFU/ml and 1x10E6 giving the highest scores (relative VAS-score 6.6 and 6.4 respectively), and placebo no 1 had an intermediate score (relative VAS-score 6.2) and placebo no 2 the lowest (relative VAS-score 5.9). The satiety scores dropped after the 3.5 hours to 3.1, 3.0, 2.5 and 2.2 respectively. The difference was small but the difference between the products persisted and an influence on satiation was noted for the probiotic product with two different concentrations of bacteria. Concerning hunger the opposite situation could be seen, i.e. the probiotic products giving a lower hunger score than the two placebo products. Directly after the meal the two probiotic product resulted in hunger scores 0.4 for both products on the VAS scale, placebo no 1 was intermediate (VAS score 0.9) and placebo no 2 gave a VAS score on hunger at 1.1. After 4 hours the figures were 6.0, 6.2, 7.0 and 6.9, respectively.

After the standard breakfast above and the four hours when data on satiety and hunger were noticed the participants were offered to eat a standard lunch meal. The participants were asked to eat until they were pleasantly satisfied and to try to get the same level of fullness after each different breakfast. After eating the probiotic products the energy intake at lunch was 3690 KJ for the product with high level of probiotics, it was 3810 for the low level probiotic. For placebo no 1 it was 3850 and for placebo no 2 it vas 3995. This shows that the probiotic products can reduce the energy intake in a postprandial meal after eating the probiotic products as part of the breakfast.

### 3) Food intake of mice

Normal flora mice of Swiss Webster strain were given acidified milk containing either LMG P-17806 or NCFB 1748 or a placebo product with the same composition but without probiotics for 10 days. The mice were between 6 and 8 weeks when the administration was started. The groups given probiotics contained 7 mice in each group and the placebo group consisted of 5 animals. The probiotic microrganisms were added to the products in a final concentration of 1x10E8 CFU/ml and the number of bacteria was stable in the product during the feeding period. The mice were given two standard daily dosages of the products, one by oral gavage feeding of 1 ml and one by sublingual injection. Both probiotic bacteria survived the passage in the gut and were present in significant numbers in the small and large intestine of the mice. Apart from the probiotic products and the placebo product the mice had *ad libitum* access to purified ingredient diet (D12450B from Research Diets Inc., New Jersey, USA). The daily food intake was compared between the two groups of mice receiving *Lactobacillus* strains versus the placebo group of mice. The products were well tolerated by the mice.

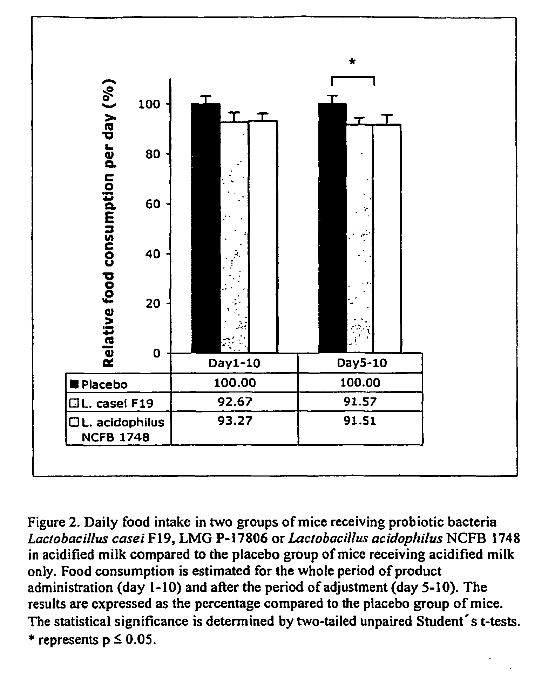

During the second part of the feeding period the food intake was significantly reduced in the groups of animals given probiotics compared to the group receiving the placebo product. See Figure 2.

### 4) Abdominal fat storage in mice

Normal flora mice were given either an acidified milk probiotic product containing LMG P-17806 or a placebo product with the same composition but without probiotics or kept as a control group given the same feed as the two other groups but without the acidified milk. The mice strain chosen was C57B16 (Charles River) which is sensitive to diet induced obesity. The number of animals included in the different groups was 15 in each of the groups given acidified milk and 3 in the control group. The mice were introduced into the study when they were 9 weeks old. The number of probiotic bacteria in the product was 1x10E8 CFU/ml and the mice were allowed to eat the product *ad libitum,* 5 days a week, for a total number of 12 weeks. For rapid weight gain the mice were fed with a high fat diet (D 12309 from Research Diets Inc. New Jersey, USA containing 36% fat for the first 5 weeks and then D 12492 (35% fat) from the same company for the remaining weeks of the trial). After 12 weeks the mice were sacrificed and the data on weight gain, food consumption and amount of abdominal fat was analysed. The mice given acidified milk product gained less weight than the control mice not receiving any acidified milk. The mice given acidified milk with probiotics also had a lower storage of abdominal fat compared to the group receiving acidified milk without probiotics and the control group not receiving any acidified milk at all.

### 5) Gene-expression in mice

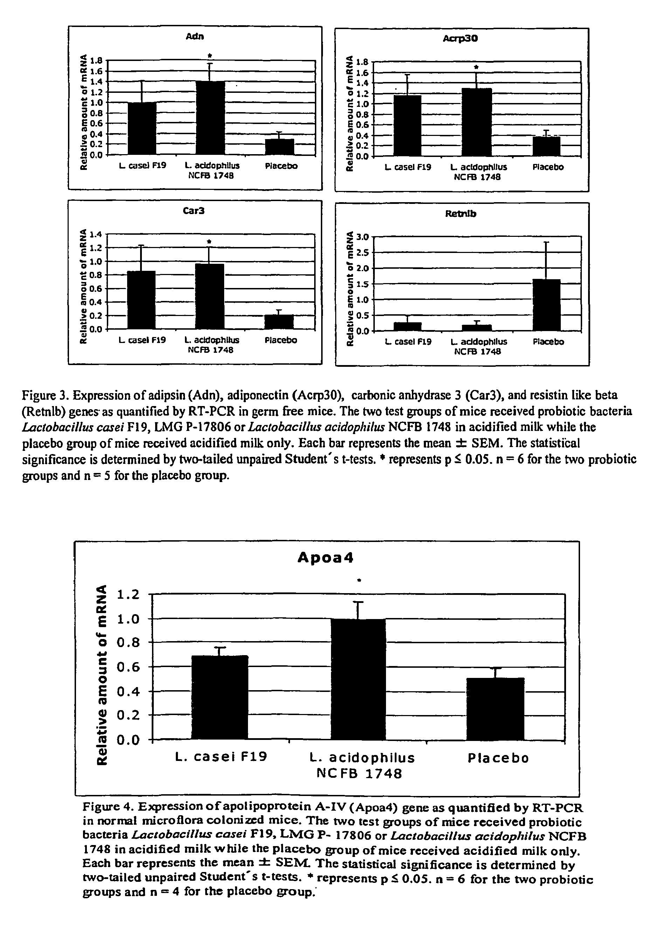

Germ free and normal flora mice of Swiss Webster strain were given acidified milk containing either LMG P-17806 or NCFB 1748 or a placebo product with the same composition but without probiotics for 10 days. The mice were between 6 and 8 weeks of age when the administration was started. The groups given probiotics contained 6 mice in each group and the placebo group consisted of 4 animals. The probiotic microrganisms were added to the products in final concentration of 1x10E8 CFU/ml and the number of bacteria was stable in the product during the feeding period. The mice were given the product daily by oral gavage feeding. During the study the mice had *ad libitum* access to purified ingredient diet (D12450B from Research Diets Inc., New Jersey, USA). Both probiotic bacteria survived the passage in the gut and were present in significant numbers in the small and large intestine of the mice. The mice were sacrificed and the gene-expression in the small intestine was analysed by oligonucleotid micro array technology. Apart from expected effects on the immune system, unexpected effects on the expression of a number of genes involved in energy metabolism and homeostasis were found to be different in the placebo group compared to the two probiotic groups. Among the genes showing a significant change in expression were Scd1, Acrp30, Adn, Thrsp, Car3, and Apoa-4, all of which were up regulated in the probiotic groups and Retnlb which was down regulated in the probiotic groups, compared to the placebo group. The pattern of differential gen-expression was very similar for the probiotic strains used. Differential expression of adipsin (Adn), adiponectin (Acrp 30), carbonic anhydrase 3 (Car3) and resistin like beta (Retnlb) genes in germ free mice was confirmed by quantitative Real- Time PCR technique, see Figure 3. Figure 4 shows the expression levels of apolipoprotein A-IV (Apoa4) in normal flora colonised mice as verified by quantitative Real-Time PCR approach.

### 6) Dosage

The total dosage of the individual strains of probiotic bacteria in the different experiments described above was 1x10E8-1x10E9 CFU (mice experiments) and 2,5x10E8-2,5x10E10 (human experiments). This dosage needs to be administrated in a portion or as a daily dose meaning that e.g. a drink for human use that is consumed in amounts 200-500 ml need to contain 0,5x10E6-1,25x10E8 CFU/ml of the individual strain for efficacy and a capsule need to contain the total amount of bacteria in approximately 1 g of the content in the capsule i.e 1x10E8-2,5x10E10. For the highest concentrations of bacteria the bacteria need to be concentrated by freeze drying or spray drying.

### 7) Preparation of product

The probiotic bacteria have to be added to products based on milk, cereals or fruits. The bacteria were added as a concentrate e.g. lyophilized and the survival has been analysed in all tree matrixes. The survival was very good in milk based and cereal based products. In fruit based products, the survival is influenced by the fruit type. As a lyophilised powder care needs to be taken concerning the water activity and exposure to oxygen. The results from product production can be seen in table 1.

The probiotic bacteria can also be added to milk based products together with other lactic acid bacteria for the production of different kinds of cultured products. In this environment the probiotic bacteria can multiply and will be well adjusted to the acid environment giving a good survival also at the low final pH in those products. The results from product production can be seen in table 1.

**Table 1. Survival of probiotic bacteria in different product matrixes measured as million CFU/g. The products were stored at 8°C.**

| Bacteria | Milk | Smoothie Milk/fruit | Cereal drink | Lyophilized powder in cereal mix | Fruit juice Black currant | Fruit juice Apple | Yoghurt drink | Yoghurt | Sour milk |
|---|---|---|---|---|---|---|---|---|---|
| LMG P-17806, day 1 | 53 | 56 | 50 | 45 | 46 | 53 | 67 | 70 | 74 |
| LMG P-17806, day 21 | 55 | 51 | 52 | 39 | 13 | 35 | 55 | 75 | 63 |
| NCFB 1748, day 1 | 50 | 53 | 56 | 43 | 50 | 55 | 57 | 56 | 60 |
| NCFB 1748, day 21 | 42 | 43 | 43 | 37 | 3 | 30 | 32 | 42 | 44 |
| Bb12, day 1 | 59 | 60 | 65 | 65 | 62 | 67 | 62 | 73 | 68 |
| Bb 12, day 21 | 49 | 47 | 60 | 31 | 14 | 49 | 51 | 68 | 60 |

### References:

1) Agerholm, 1., M.I. Bell, G.k. Grunwald and A. Astrup. 2000. The effect of a probiotic milk product on plasma cholesterol: a meta-analysis of short-term intervention studies. Eur. J. clin. Nutr. 54:856-860.
2) Ali A. A., I. A. Mohamed, C. T. Hansen, T. Wang, M. T. Velasquez and S. J. Bhathena. 2002. Effect of probiotics and isoflavones on metabolic parameters in a genetic model of obesity and diabetes. FASEB J. 16:p A1014.
3) Ali. A. A., M. T. Velasquez, C. T. Hansen, A. I. Mohamed, and S. J. Bhathena. 2004. Effect of soybean isoflavones, probiotics and their interactions on the metabolism and endocrine system in an animal model of obesity and diabetes. J. Nutritional Bioch. 15:583-590.
4) Bojrab G.. 2000. Composition and method for treatment of gastrointestinal disorders and hyperlipedemia. US patent 6 696 057
5) Bojrab G.. 2000. Composition for treatment of obesity. US patent 6 641 808.
6) Bojrab G.. 2001. Composition, of L. bulgaricus and S. thermophilus, for the treatment of gastrointestinal disorders, hyperlipidemia, autoimmune diseases, and obesity. European patent application 1 177 794.
7) Bäckhed F., H Ding, T. Wang, L. V. Hooper, G. Y. Koh, A. Nagy, C. F. Semenkovich and J. I. Gordon. 2004. The gut microbiota as an environmental factor that regulates fat storage. PNAS 101:15718-15723.
8) Di Caro S., H. Tao, A. Grillo, C. Elia, G. Gasbarrini, A. R. Sepulveda, and A. Gasbarrini. 2005. Effects of Lactobacillus GG on genes expression pattern in small bowel mucosa. Digestive and Liver Disease 37:320-329.
9) Gorsek W. F. 2000. Weight loss composition containing green tea, hydroxycitric acid, 5-hydroxytryptophane, glucomannan, picolinate and Lactobacillus. US patent 6 383 482.
10) Gorsek W. F.. 2002. Thermogenic weight management composition. US patent 6 565 847.
11) Hooper L. V., M Wong, A. Thelin, L. Hansson, P.G. Falk and J.I. Gordon. 2001. Molecular analysis of commensal host-microbial relationship in the intestine. Science 291 (5505):881-884.
12) Park H. O., Y. B. Bang, H. J. Joung, B. C. Kim and H. R. Kim. 2004. Lactobacillus KTCK 0774BP and acetobacter KCTC 0773BP for treatment or prevention of obesity and diabetes mellitus. US patent 6 808 703.
13) Pereira D. I. and G. R. Gibson. 2002. Effects of consumption of probiotics and prebiotics on serum lipid levels in humans. Crit. Rev. Biochem Mol Biol. 37:259-281.
14) Steppan C. M., S. T. Balley, S. Bath, E. J. Brown, R. R. Banerjee, C. M. Wright, H. R. Patel, R. S. Ahlma, and M. A. Lazar. 2001. The hormone resistine links obesity to diabetes. Nature 409:307.
15) Song S.H., S. K. Kang, j. H. Kim, y. H. Park, and H. O. Park 2002. Microorganisms for ihibiting obesity and diabetes mellitus. PCT patent application WO 04/014403.
16) Yanagida F., and K. Sano. 2000. Obesity preventative food. Japanese patent application 2001-292728.
17) Tabuchi M., Ozaki M., Tamura A., Yamada N., Ishida T., Hosoda M., Hosono A. 2003. Antidiabetic effect of Lactobacillus GG in streptozotocin-induced diabetic rats. Biosci Biotechnol Biochem. Jun;67(6):1421-4.
18) Kalavathy R., Abdullah N., Jalaludin S., Ho Y.W. 2003. Effects of Lactobacillus cultures on growth performance, abdominal fat deposition, serum lipids and weight of organs of broiler chickens. Br Poult Sci. Mar;44(1):139-44.
19) Matsuzaki T., Nagata Y., Kado S., Uchida K., Kato I., Hashimoto S, Yokokura T. 1997. Prevention of onset in an insulin-dependent diabetes mellitus model, NOD mice, by oral feeding of Lactobacillus casei. APMIS. Aug;105(8):643-920)
20) Ohlson K., Björneholm S., Fonden R., Svensson U., 2002. Lactobacillus F19: A probiotic strain suitable for consumer products. Microbial ecology in health and disease. Vol 14, supplement 3:27-32.
21) Sullivan A., Barkholt L., Nord C.E. 2003. Lactobacillus acidophilus, Bifidobacterium lactis and Lactobacillus F19 prevent antibiotic-associated ecological disturbances of Bacteroides fragilis in the intestine.J Antimicrob Chemother Aug;52(2):308-11.

## Claims

1. Use of at least one probiotic bacteria for the manufacture of a probiotic product for lowering weight gain, wherein the at least one probiotic bacteria is selected from the group consisting of *Lactobacillus casei* F19 (LMG P-17806), *Lactobacillus acidophilus* (NCFB1748) and *Bifidobacterium lactis* Bb12 (DSM 15954).

2. Use of at least one probiotic bacteria for the manufacture of a probiotic product for increasing satiety, wherein the at least one probiotic bacteria is selected from the group consisting of Lactobacillus casei F19 (LMG P-17806), Lactobacillus acidophilus (NCFB 1748), Bifidobacterium lactis Bb12 (DSM 15954).

3. Use of at least one probiotic bacteria for the manufacture of a probiotic product for prolonging satiation, wherein the at least one probiotic bacteria is selected from the group consisting of Lactobacillus casei F19 (LMG P-17806), Lactobacillus acidophilus (NCFB 1748), Bifidobacterium lactis Bb12 (DSM 15954).

4. Use of at least one probiotic bacteria for the manufacture of a probiotic product for reducing food intake, wherein the at least one probiotic bacteria is selected from the group consisting of Lactobacillus casei F19 (LMG P-17806), Lactobacillus acidophilus (NCFB 1748), Bifidobacterium lactis Bb12 (DSM 15954).

5. Use of at least one probiotic bacteria for the manufacture of a probiotic product for the up-regulation of the expression of a gene selected from the group consisting of Scd1, adiponectin (Acrp 30), adipsin (Adn), Thrsp, carbonic anhydrase 3 (Car3) and apolipoprotein A-IV (Apoa4), wherein the at least one probiotic bacteria is selected from the group consisting of Lactobacillus casei F19 (LMG P-17806), Lactobacillus acidophilus (NCFB 1748), Bifidobacterium lactis Bb12 (DSM 15954).

6. Use of at least one probiotic bacteria for the manufacture of a probiotic product for enhancing insulin sensitivity or treating insulin insensitivity, wherein the enhanced insulin sensitivity is due to a down-regulation of the expression of resistin like beta (Retnlb), wherein the at least one probiotic bacteria is selected from the group consisting of Lactobacillus casei F19 (LMG P-17806), Lactobacillus acidophilus (NCFB 1748), Bifidobacterium lactis Bb12 (DSM 15954).

7. Use of a at least one probiotic bacteria for the manufacture of a probiotic product for decreasing the risk for developing a metabolic syndrome including obesity, abdominal fat deposition and type II diabetes, wherein the at least one probiotic bacteria is selected from the group consisting of *Lactobacillus casei* F19 (LMG P-17806), *Lactobacillus acidophilus* (NCFB 1748), *Bifidobacterium lactis* Bb12 (DSM 15954).

8. Use of at least one probiotic bacteria for the manufacture of a probiotic product for lowering storage of abdominal fat, wherein the at least one probiotic bacteria is selected from the group consisting of *Lactobacillus casei* F19 (LMG P-17806), *Lactobacillus acidophilus* (NCFB1748) and *Bifidobacterium lactis* Bb12 (DSM 15954).

9. Use according to any of claims 1-8, **characterized in that** the probiotic bacteria is used for manufacturing of a product selected from the group consisting of a product based on milk, a product based on cereal and a product based on fruit or compositions derived thereof.

10. Use according to any of claims 1-9, **characterized in that** the probiotic bacteria is used as a concentrate e.g. lyophilized.

11. Use according to any of claims 1-10, **characterized in that** the probiotic bacteria is comprised in a capsule.

12. Use according to claim 11, **characterized in that** the capsule contains a dosage of probiotic bacteria between 1x10⁸ CFU to 2.5x10⁸ CFU.

13. Use according to any of claims 1-12, wherein the probiotic product is selected from the group consisting of a food product, feed product, dietary supplement, natural remedy and pharmaceutical active formulation.

14. A probiotic product comprising at least one probiotic bacteria selected from the group consisting of *Lactobacillus casei* F19 (LMG P-17806), *Lactobacillus acidophilus* (NCFB 1748) and *Bifidobacterium lactis* Bb12 (DSM 15954) for use in lowering weight gain, increasing satiety, prolonging satiation, reducing food intake or lowering storage of abdominal fat.

15. A probiotic product comprising at least one probiotic bacteria selected from the group consisting of *Lactobacillus casei* F19 (LMG P-17806), *Lactobacillus acidophilus* (NCFB 1748) and *Bifidobacterium lactis* Bb12 (DSM 15954) for use in up-regulation of the expression of a gene selected from the group consisting of Scd1, adiponectin (Acrp 30), adipsin (Adn), Thrsp, carbonic anhydrase 3 (Car3) and apolipoprotein A-IV (Apoa4).

16. A probiotic product comprising at least one probiotic bacteria selected from the group consisting of *Lactobacillus casei* F19 (LMG P-17806), *Lactobacillus acidophilus* (NCFB 1748) and *Bifidobacterium lactis* Bb12 (DSM 15954) for use in enhancing insulin sensitivity or treating insulin insensitivity, wherein the enhanced insulin sensitivity is due to a down-regulation of the expression of resistin like beta (Retnlb).

17. A probiotic product comprising at least one probiotic bacteria selected from the group consisting of *Lactobacillus casei* F19 (LMG P-17806), *Lactobacillus acidophilus* (NCFB 1748) and *Bifidobacterium lactis* Bb12 (DSM 15954) for use in decreasing the risk for developing a metabolic syndrome including obesity, abdominal fat deposition and type II diabetes.

## Patentansprüche

1. Verwendung mindestens einer probiotischen Bakterie zur Herstellung eines probiotischen Produkts zur Verringerung der Gewichtszunahme, wobei die mindestens eine probiotische Bakterie ausgewählt ist aus der Gruppe, bestehend aus *Lactobacillus casei* F19 (LMG P-17806), *Lactobacillus acidophilus* (NCFB 1748) und *Bifidobacterium lactis* Bb12 (DSM 15954).

2. Verwendung mindestens einer probiotischen Bakterie zur Herstellung eines probiotischen Produkts zur Erhöhung der Sättigung, wobei die mindestens eine probiotische Bakterie ausgewählt ist aus der Gruppe, bestehend aus *Lactobacillus casei* F19 (LMG P-17806), *Lactobacillus acidophilus* (NCFB 1748) und *Bifidobacterium lactis* Bb12 (DSM 15954).

3. Verwendung mindestens einer probiotischen Bakterie zur Herstellung eines probiotischen Produkts zur Verlängerung des Sättigungsgefühls, wobei die mindestens eine probiotische Bakterie ausgewählt ist aus der Gruppe, bestehend aus *Lactobacillus casei* F19 (LMG P-17806), *Lactobacillus acidophilus* (NCFB 1748) und *Bifidobacterium lactis* Bb12 (DSM 15954).

4. Verwendung mindestens einer probiotischen Bakterie zur Herstellung eines probiotischen Produkts zur Senkung der Nahrungsaufnahme, wobei die mindestens eine probiotische Bakterie ausgewählt ist aus der Gruppe, bestehend aus *Lactobacillus casei* F19 (LMG P-17806), *Lactobacillus acidophilus* (NCFB 1748) und *Bifidobacterium lactis* Bb12 (DSM 15954).

5. Verwendung mindestens einer probiotischen Bakterie zur Herstellung eines probiotischen Produkts zur Up-Regulation der Expression eines Gens, ausgewählt aus der Gruppe, bestehend aus Scd1, Adiponectin (Acrp 30), Adipsin (Adn), Thrsp, Carboanhydrase 3 (Car3) und Apolipoprotein A-IV (Apoa4), wobei die mindestens eine probiotische Bakterie ausgewählt ist aus der Gruppe, bestehend aus *Lactobacillus casei* F19 (LMG P-17806), *Lactobacillus acidophilus* (NCFB 1748) und *Bifidobacterium lactis* Bb12 (DSM 15954).

6. Verwendung mindestens einer probiotischen Bakterie zur Herstellung eines probiotischen Produkts zur Erhöhung der Insulinempfindlichkeit oder zur Behandlung der Insulinunempfindlichkeit, wobei die erhöhte Insulinempfindlichkeit auf eine Down-Regulation der Expression von Resistin-ähnlichem Beta (Retnlb) zurückzuführen ist, wobei die mindestens eine probiotische Bakterie ausgewählt ist aus der Gruppe, bestehend aus *Lactobacillus casei* F19 (LMG P-17806), *Lactobacillus acidophilus* (NCFB 1748) und *Bifidobacterium lactis* Bb12 (DSM 15954).

7. Verwendung mindestens einer probiotischen Bakterie zur Herstellung eines probiotischen Produkts zur Senkung des Risikos, ein Stoffwechselsyndrom, einschließlich Adipositas, Fettablagerungen im Abdominalbereich und Typ-II-Diabetes, zu entwickeln, wobei die mindestens eine probiotische Bakterie ausgewählt ist aus der Gruppe, bestehend aus *Lactobacillus casei* F19 (LMG P-17806), *Lactobacillus acidophilus* (NCFB 1748) und *Bifidobacterium lactis* Bb12 (DSM 15954).

8. Verwendung mindestens einer probiotischen Bakterie zur Herstellung eines probiotischen Produkts zur Verringerung der Speicherung von Fett im Abdominalbereich, wobei die mindestens eine probiotische Bakterie ausgewählt ist aus der Gruppe, bestehend aus *Lactobacillus casei* F19 (LMG P-17806), *Lactobacillus acidophilus* (NCFB 1748) und *Bifidobacterium lactis* Bb12 (DSM 15954).

9. Verwendung nach einem der Ansprüche 1-8, **dadurch gekennzeichnet, dass** die probiotische Bakterie zur Herstellung eines Produkts verwendet wird, ausgewählt aus der Gruppe, bestehend aus einem Produkt auf Milchbasis, einem Produkt auf Getreidebasis und einem Produkt auf Obstbasis oder davon abgeleiteten Zusammensetzungen.

10. Verwendung nach einem der Ansprüche 1-9, **dadurch gekennzeichnet, dass** die probiotische Bakterie als ein Konzentrat verwendet wird, beispielsweise lyophilisiert.

11. Verwendung nach einem der Ansprüche 1-10, **dadurch gekennzeichnet, dass** die probiotische Bakterie in einer Kapsel enthalten ist.

12. Verwendung nach Anspruch 11, **dadurch gekennzeichnet, dass** die Kapsel eine Dosis der probiotischen Bakterie zwischen 1x10⁸ CFU und 2,5x10⁸ CFU enthält.

13. Verwendung nach einem der Ansprüche 1-12, wobei das probiotische Produkt ausgewählt ist aus der Gruppe, bestehend aus einem Lebensmittelprodukt, einem Futterprodukt, einem Nahrungsergänzungsmittel, einem Naturheilmittel und einer pharmazeutisch wirksamen Formulierung.

14. Probiotisches Produkt, umfassend mindestens eine probiotische Bakterie, ausgewählt aus der Gruppe, bestehend aus *Lactobacillus casei* F19 (LMG P-17806), *Lactobacillus acidophilus* (NCFB 1748) und *Bifidobacterium lactis* Bb12 (DSM 15954) zur Verwendung zur Verringerung der Gewichtszunahme, zur Erhöhung der Sättigung, zur Verlängerung des Sättigungsgefühls, zur Senkung der Nahrungsaufnahme und zur Verringerung der Speicherung von Fett im Abdominalbereich.

15. Probiotisches Produkt, umfassend mindestens eine probiotische Bakterie, ausgewählt aus der Gruppe, bestehend aus *Lactobacillus casei* F19 (LMG P-17806), *Lactobacillus acidophilus* (NCFB 1748) und *Bifidobacterium lactis* Bb12 (DSM 15954) zur Verwendung zur Up-Regulation der Expression eines Gens, ausgewählt aus der Gruppe, bestehend aus Scd1, Adiponectin (Acrp 30), Adipsin (Adn), Thrsp, Carboanhydrase 3 (Car3) und Apolipoprotein A-IV (Apoa4).

16. Probiotisches Produkt, umfassend mindestens eine probiotische Bakterie, ausgewählt aus der Gruppe, bestehend aus *Lactobacillus casei* F19 (LMG P-17806), *Lactobacillus acidophilus* (NCFB 1748) und *Bifidobacterium lactis* Bb12 (DSM 15954) zur Verwendung zur Erhöhung der Insulinempfindlichkeit oder zur Behandlung der Insulinunempfindlichkeit, wobei die erhöhte Insulinempfindlichkeit auf einer Down-Regulation der Expression von Resistin-ähnlichem Beta (RetnIb) zurückzuführen ist.

17. Probiotisches Produkt, umfassend mindestens eine probiotische Bakterie, ausgewählt aus der Gruppe, bestehend aus *Lactobacillus casei* F19 (LMG P-17806), *Lactobacillus acidophilus* (NCFB 1748) und *Bifidobacterium lactis* Bb12 (DSM 15954) zur Verwendung zur Senkung des Risikos, ein Stoffwechselsyndrom, einschließlich Adipositas, Fettablagerungen im Abdominalbereich und Typ-II-Diabetes, zu entwickeln.

## Revendications

1. Utilisation d'au moins une bactérie probiotique pour la fabrication d'un produit probiotique destiné à réduire la prise de poids, dans laquelle l'au moins une bactérie probiotique est choisie dans le groupe consistant en *Lactobacillus casei* F19 (LMG P-17806), *Lactobacillus acidophilus* (NCFB1748) et *Bifidobacterium lactis* Bb12 (DSM 15954).

2. Utilisation d'au moins une bactérie probiotique pour la fabrication d'un produit probiotique destiné à accroître la satiété, dans laquelle l'au moins une bactérie probiotique est choisie dans le groupe consistant en *Lactobacillus casei* F19 (LMG P-17806), *Lactobacillus acidophilus* (NCFB 1748), *Bifidobacterium lactis* Bb12 (DSM 15954).

3. Utilisation d'au moins une bactérie probiotique pour la fabrication d'un produit probiotique destiné à prolonger la satiation, dans laquelle l'au moins une bactérie probiotique est choisie dans le groupe consistant en *Lactobacillus casei* F19 (LMG P-17806), *Lactobacillus acidophilus* (NCFB 1748), *Bifidobacterium lactis* Bb12 (DSM 15954).

4. Utilisation d'au moins une bactérie probiotique pour la fabrication d'un produit probiotique destiné à réduire l'apport alimentaire, dans laquelle l'au moins une bactérie probiotique est choisie dans le groupe consistant en *Lactobacillus casei* F19 (LMG P-17806), *Lactobacillus acidophilus* (NCFB 1748), *Bifidobacterium lactis* Bb12 (DSM 15954).

5. Utilisation d'au moins une bactérie probiotique pour la fabrication d'un produit probiotique destiné à réguler à la hausse l'expression d'un gène choisi dans le groupe consistant en Scd1, adiponectine (Acrp 30), adipsine (Adn), Thrsp, anhydrase carbonique 3 (Car3) et apolipoprotéine A-IV (Apoa4), dans laquelle l'au moins une bactérie probiotique est choisie dans le groupe consistant en *Lactobacillus casei* F19 (LMG P-17806), *Lactobacillus acidophilus* (NCFB 1748), *Bifidobacterium lactis* Bb12 (DSM 15954).

6. Utilisation d'au moins une bactérie probiotique pour la fabrication d'un produit probiotique destiné à améliorer la sensibilité à l'insuline ou traiter l'insensibilité à l'insuline, dans laquelle la sensibilité à l'insuline améliorée est due à une régulation à la baisse de l'expression du gène resistin like beta (Retnlb), dans laquelle l'au moins une bactérie probiotique est choisie dans le groupe consistant en *Lactobacillus casei* F19 (LMG P-17806), *Lactobacillus acidophilus* (NCFB 1748), *Bifidobacterium lactis* Bb12 (DSM 15954).

7. Utilisation d'au moins une bactérie probiotique pour la fabrication d'un produit probiotique destiné à réduire le risque de développer un syndrome métabolique incluant une obésité, un dépôt de graisse abdominale et un diabète de type 2, dans laquelle l'au moins une bactérie probiotique est choisie dans le groupe consistant en *Lactobacillus casei* F19 (LMG P-17806), *Lactobacillus acidophilus* (NCFB 1748), *Bifidobacterium lactis* Bb12 (DSM 15954).

8. Utilisation d'au moins une bactérie probiotique pour la fabrication d'un produit probiotique destiné à réduire le stockage de graisse abdominale, dans laquelle l'au moins une bactérie probiotique est choisie dans le groupe consistant en *Lactobacillus casei* F19 (LMG P-17806), *Lactobacillus acidophilus* (NCFB1748) et *Bifidobacterium lactis* Bb12 (DSM 15954).

9. Utilisation selon l'une quelconque des revendications 1 à 8, **caractérisée en ce que** la bactérie probiotique est utilisée pour la fabrication d'un produit choisi dans le groupe consistant en un produit à base de lait, un produit à base de céréales et un produit à base de fruits ou des compositions dérivées de ceux-ci.

10. Utilisation selon l'une quelconque des revendications 1 à 9, **caractérisée en ce que** la bactérie probiotique est utilisée sous forme de concentré, par exemple lyophilisée.

11. Utilisation selon l'une quelconque des revendications 1 à 10, **caractérisée en ce que** la bactérie probiotique est comprise dans une capsule.

12. Utilisation selon la revendication 11, **caractérisée en ce que** la capsule contient une dose de bactérie probiotique comprise entre 1x10⁸ UFC et 2,5x10⁸ UFC.

13. Utilisation selon l'une quelconque des revendications 1 à 12, dans laquelle le produit probiotique est choisi dans le groupe consistant en un produit alimentaire, un produit d'alimentation animale, un complément alimentaire, un remède naturel et une formulation active pharmaceutique.

14. Produit probiotique comprenant au moins une bactérie probiotique choisie dans le groupe consistant en *Lactobacillus casei* F19 (LMG P-17806), *Lactobacillus acidophilus* (NCFB 1748) et *Bifidobacterium lactis* Bb12 (DSM 15954) destiné à être utilisé pour réduire la prise de poids, accroître la satiété, prolonger la satiation, réduire l'apport alimentaire ou réduire le stockage de graisse abdominale.

15. Produit probiotique comprenant au moins une bactérie probiotique choisie dans le groupe consistant en *Lactobacillus casei* F19 (LMG P-17806), *Lactobacillus acidophilus* (NCFB 1748) et *Bifidobacterium lactis* Bb12 (DSM 15954) destiné à être utilisé pour réguler à la hausse l'expression d'un gène choisi dans le groupe consistant en Scd1, adiponectine (Acrp 30), adipsine (Adn), Thrsp, anhydrase carbonique 3 (Car3) et apolipoprotéine A-IV (Apoa4).

16. Produit probiotique comprenant au moins une bactérie probiotique choisie dans le groupe consistant en *Lactobacillus casei* F19 (LMG P-17806), *Lactobacillus acidophilus* (NCFB 1748) et *Bifidobacterium lactis* Bb12 (DSM 15954) destiné à être utilisé pour améliorer la sensibilité à l'insuline ou traiter l'insensibilité à l'insuline, dans lequel la sensibilité à l'insuline améliorée est due à une régulation à la baisse de l'expression du gène resistin like beta (Retnlb).

17. Produit probiotique comprenant au moins une bactérie probiotique choisie dans le groupe consistant en *Lactobacillus casei* F19 (LMG P-17806), *Lactobacillus acidophilus* (NCFB 1748) et *Bifidobacterium lactis* Bb12 (DSM 15954) destiné à être utilisé pour réduire le risque de développer un syndrome métabolique incluant une obésité, un dépôt de graisse abdominale et un diabète de type 2.
